# EUROPEAN PATENT SPECIFICATION

(11) **EP 0 602 137 B1**
(45) Date of publication and mention of the grant of the patent: **23.01.2002**
(21) Application number: 92919047.8
(22) Date of filing: 07.09.1992
(51) Int. Cl.: A23L 1/303, A23L 1/275, A23L 1/035, A23L 1/05

(54) **CAROTENOID COMPOSITION**
CAROTINOIDE ZUSAMMENSETZUNG
COMPOSITION A BASE DE CAROTENOIDE

(30) Priority: 06.09.1991 AU 825891
(43) Date of publication of application: 22.06.1994
(73) Proprietor: Cognis Australia Pty Ltd, Broadmeadows, Victoria 3047 (AU)
(72) Inventor: SCHLIPALIUS, Lance Elliott, Cheltenham, VIC 3192 (AU)
(74) Representative: Barrett-Major, Julie Diane
(86) International application number: AU9200470
(87) International publication number: WO9304598

(56) References cited:
- AU-A- 1 070 888
- AU-A- 7 062 887
- GB-A- 985 613
- GB-A- 2 248 170
- US-A- 3 734 745
- PATENT ABSTRACTS OF JAPAN vol. 15, no. 252 (C-0844) 26 June 1991 & JP-A-03 081 230 (OTSUKA SHOKUHIN KK) 5 April 1991
- FOOD MANUFACTURE, vol.61, no.12, 1986 pages 40 - 41 R.SEAL 'Facing up to the "additives" challenge'
- PATENT ABSTRACTS OF JAPAN vol. 7, no. 66 (C-157) 18 March 1983 & JP-A-58 001 752 (TOYO INK SEIZO KK) 7 January 1983
- DERWENT ABSTRACT Accession No. 91-118018/17, Class D13 E24; & CN,A,1044478 (SALT REARCH INST) 8 August 1990 (08.08.90), Title.
- DERWENT ABSTRACT Accession No. 21581K/09, Class D13 E24; & JP,A,58 013 367 (TOYO INK MFG KK) 25 January 1983 (25.01.83), Abstract.
- DERWENT ABSTRACT Accession No. 86834B/48, Class D13 E24; & JP,A,54 135 822 (KAWAI SEIYAKU KK) 22 October 1979 (22.10.79), Abstract.

## Description

The invention relates to a carotenoid composition derived from all natural sources, in particular the composition is an emulsion or dried product and a process for producing an all natural carotenoid composition.

Carotenoids are generally only soluble to any extent in lipid materials or organic solvents. Many of their applications for use, however, make it desirable that they are contained in aqueous based systems for the purposes of providing a safe source of vitamin A, colourisation (particularly food products which are generally in aqueous based materials) or as a powdered product in special preparations which are based on aqueous materials.

To overcome the virtual insolubility of carotenoids in water, so called water-dispersible forms of carotenoid products are manufactured for use. They are a significant part of the economic market for carotenoids.

Recently, there has been a consumer driven preference for natural products in the market place and this has also been true for carotenoid products. The demand for products containing natural beta-carotene (a carotenoid) has grown substantially over the last five years. Natural beta-carotene is derived from certain animals, plants and micro-organisms, such as the algae Dunaliella salina. However, it is inconsistent in the eyes of consumers to make a water dispersible natural carotenoid product using, for example, beta-carotene extracted from Dunaliella salina, and then to use synthetic raw materials in the manufacture of the water-dispersible material.

Water dispersible carotenoid compositions are described, for example, in United States Patents Nos. 3,734,745 and 4,397,878, British Patent No. 985,613, and Australian Patent No. 10708/88. However, these compositions rely on the incorporation of synthetic ingredients in order to achieve the water-dispersibility of the carotenoid.

Consequently, there is a need to provide a completely natural product of water dispersible carotenoids in both a liquid emulsion and dried product form which represent the main forms in which these products are sold.

Emulsions are stable mixtures of two or more immiscible liquids held in suspension by stabilizers and small percentages of substances called emulsifiers.

Certain emulsions occur naturally. These include milk, cream, avocado, egg yolk and nuts. These emulsions contain fine dispersions of natural lipid material which are stabilized in two ways. First, by small sized droplets of fat which occur within the cell structure and cannot coalesce into larger particles due to the solidity or very high viscosity of the product. Avocado and nuts are examples of this type of emulsion. Secondly, in more fluid products with reduced viscosity, the small fat droplets are stabilized within the aqueous structure by naturally present emulsifying agents or biochemicals and by polymeric molecules of carbohydrates, proteins and lipids or combinations of all three often where the molecules are chemically bonded together which restrict the coalescing of the small lipid globules.

In more recent times these stabilizing agents have been extracted from the natural materials to provide concentrated sources of emulsifiers and stabilizers which can be used more generally for food and other biological emulsions. For example, in the preparation of salad dressing products or mayonnaise, starches, egg yolk, microbial and vegetable isolates have been selected to assist in stablizing vegetable oils to provide a pleasing mouth texture and flavour for culinary purposes. Although the natural materials are quite satisfactory for many applications there have also been chemically modified stabilizers and emulsifiers produced which can make more stable emulsions. The chemically modified stabilizers and emulsifiers have the disadvantage that they are produced from synthetic chemical processes and for that reason they are not favoured by certain manufacturers of food products.

Previous emulsions of carotenoids have normally incorporated these chemically modified stabilizing agents for the production of carotenoid emulsions and dried water dispersible products.

It is a general requirement that carotenoids be protected against oxidation and that the liquid preparations be preserved against microbiological spoilage. For these reasons, synthetic antioxidants and synthetic preservatives have also been used in carotenoid liquid emulsions and dried powders.

The object of the invention is to provide a carotenoid composition derived principally from natural sources and a method of preparing these compositions employing a natural emulsion system to create oil in water dispersions. To achieve this result, certain mechanical and physical processes are used to provide a complete process such that a water dispersible powder and liquid emulsion product can be produced without the need for synthetic or unnatural ingredients at all.

According to this invention, there is provided a carotenoid composition comprising:
a) a carotenoid in an oil solvent;
b) a dispersion of a water-dispersible matrix and a stabilizer, and optionally a non-oil solvent; and
c) an emulsifier,
and which composition is substantially free of synthetic or chemically-modified components, and wherein the weight of the carotenoid in the carotenoid composition when dried is from 0.5 up to 12wt %.

According to another form of the invention there is provided a process for producing a carotenoid composition comprising the following steps:
(a) forming an oil phase by dispersing a carotenoid in an oil solvent;
(b) forming an aqueous phase by dispersing a water dispersible matrix, a stabilizier and a non-oil solvent;
(c) admixing the oil phase and the aqueous phase; and
(d) emulsifying the admixture with an emulsifier,
provided that the carotenoid composition so formed is substantially free of synthetic or chemically modified components.

In a preferred form of the invention, the carotenoid composition is homogenized to help reduce the size of the oil globules in the emulsion which improves the stability and particularly helps to stop ringing of the colour at the surface of, for example beverage products.

In a further preferred form of the invention, the carotenoid compsoition is dried and comprises between 0.5% to 12% by weight of carotenoid and preferably between 7.5% to 12% by weight of carotenoid. The dried or powdered form of the invention has particular uses in, for example, cake mixes and as drink powders and in some instances is preferred by users than the non-dried form. In this form the non-oil solvent of the emulsion is removed and the oil globules containing the carotenoid and the oil solvent are trapped in a solid continuous phase of the water dispersible matrix and stabilizer.

In the dried form, the water dispersible matrix preferably comprises up to 90% by weight and more preferably 55% to 75% of the carotenoid composition.

In three preferred forms of the invention, when the carotenoid composition is an emulsion, the emulsion comprises up to 7.5%, between 0.1% and 6% and between 2% and 5% by weight of carotenoid. Preferably, the carotenoid is selected from fat soluble retinoids, beta-carotene, lutein, lycopene, astaxanthin, canthaxanthin, phytoene, alpha-carotene, apo carotenal, retinol, capsanthin, oleo resin paprika, zeaxanthin, beta-cryptoxanthin, phytofluene, gamma-carotene and natural carotenoid isolates and mixtures thereof.

In another form of the invention the water dispersible matrix is comprised of a non-oil solvent and any of a non-oil solvent soluble matrix and a non-oil solvent soluble anti-oxidant. In the preferred forms of the invention the solvent soluble matrix is a combination of any one or more of sucrose, glucose, fructose, mannose, pentoses, maltose and malto dextrins (referred to hereinafter as sugar (matrix)), the non-oil solvent is selected from water, glycerol and mixtures thereof and the non-oil solvent soluble anti-oxidant is a combination of any one or more of natural ascorbic acid from rosehip or acerola.

In yet another preferred form of the invention, the oil solvent contains an oil soluble antioxidant that either exists naturally or is derived from natural sources without the use of chemical or synthesized compounds, nor chemically modified using processes of synthetic chemistry. The oil soluble anti-oxidant comprises 0.1% to 2% and more preferably 0.2% to 1% by weight of the carotenoid composition. Typically the anti-oxidant is a combination of natural mixed tocopherols and other plant extracts having anti-oxidant activity. More preferably, the plant extract is selected from rosemary, eucalyptus and olive.

Preferably the oil solvent is selected from animal, vegetable or mineral oils including but not limited to soyabean, peanut, lard, olive, corn, coconut, fish, terpenoid and essential oils. Preferably these oils should be cold pressed.

The emulsifier preferably comprises 0.01% to 3% and more preferably 0.03% to 1% by weight of the carotenoid composition and is selected from extracts of animals and plants, phospholipid extracts of natural products, saponins, lecithins, milk isolates and egg products including egg yolks.

Preferably the invention further comprises a stabilizer which comprises 1% to 30% and preferably 5% to 25% by weight of the carotenoid composition. Typically the stabilizer is selected from hydrocolloids derived from natural plant and animal sources; guar, xanthan, carob, karaya, arabic, acacia and furcellaran gums; and carrageenans, alginates, celluloses, gelatins, starches, amylose, amylopectin, caseins and caseinates.

In yet a further preferred embodiment of the invention the carotenoid composition further comprises an acidulant to assist microbiological stability. The acidulant either exists naturally or is derived from natural sources without the use of chemical or synthesized compounds, nor chemically modified using processes of synthetic chemistry. Typically these are selected from naturally derived acids including citric, tartaric, ascorbic, maleic, lactic and fruit juices and other vegetable sources containing acids. The acidulant is added to the aqueous phase to adjust the pH of the aqueous phase to within the range of 2 to 5.5 and more preferably within the range 3.5 to 4.0 which assists the microbiological stability of the composition.

Preferably the carotenoid, oil solvent, water dispersible matrix, stabilizer, emulsifier, anti-oxidants and acidulant in the process for producing a carotenoid composition are also included in the groups outlined above.

In a further preferred form of the invention the process for producing a carotenoid composition includes the step of homogenizing the emulsion to obtain a fine particle structure preferably within the range 0.01 and 10 microns and more preferably within the range 0.1 to 2.0 microns. Homogenization helps to reduce the size of the oil globules in the emulsion which improves its stability and in particular helps to stop ringing of the colour at the surface of, for example, beverage products.

Preferably, the process further comprises the step of heating the admixture of the oil phase the aqueous phase prior to emulsification to a temperature of between 60°C and 150°C and holding the admixture at this temperature for sufficient time to assist the microbiological control of the product (for longer term storage). Thereafter, emulsification may take place. Typically, the material is then packed, and preferably aseptically filled into containers.

In yet a further form of the invention, the process includes the step of drying the emulsion to form a powder and preferably, the emulsion is dried using a spray drier. The dried form has particular preferred uses as described above.

In another form of the invention a carotenoid composition is provided obtainable by the invention processes.

In yet a final preferred form of the invention a process to produce a composition is provided which comprises the steps of:
(a) forming an oil phase by dispersing the carotenoid in the oil solvent;
(b) forming an aqueous phase by dispersing the water dispersible matrix, the stabilizer and the non-oil solvent;
(c) admixing the oil phase and the aqueous phase; and
(d) emulsifying the admixture with the emulsifier.

References in this specification to a particular group of components is a reference both to each individual component and to any combination of those components.

As indicated above, this invention uses only natural materials as the source of carotenoids, emulsifiers, solvent soluble matrices, stabilizers, anti-oxidants, acidulants and solvents and uses a physical technique for microbiological preservation rather than a chemical technique.

### ILLUSTRATIONS OF THE INVENTION

The invention is illustrated by the following examples.

### Example 1

An aqueous dispersion of sucrose, gum acacia, citric acid, egg yolk powder and acerola powder is created in water. A dispersion of the oil based mixture containing the solubilized carotenoid in soya bean oil is mixed with natural tocopherols as the anti-oxidant and is dispersed into the aqueous phase. The material is finely emulsified at temperatures between 50° and 80°C, subsequently homogenized to obtain a fine particle structure of between 0.01 and 10.0 microns and preferably between 0.1 and 2.0 microns. This emulsion is then held at a temperature of 75°C for sufficient time to render the microbiological load satisfactory for longer term storage. The material is then packed into suitable containers which are hermetically sealed. The product in the container is then cooled to ambient temperature.

### Example 2

The same procedure and conditions in example 1 and further, the emulsion is dried on a spray drier to form a microencapsulated or similar finished product which as a powder is stable to oxidation and microbiological spoilage and is packaged in a suitable reduced oxygen permeability package to maintain stability of the carotenoid over a period of time of about six to twelve months.

### Example 3

A typical emulsion and dried product composition is:

| | **Emulsion** | | **Dried Product** |
|---|---|---|---|
| | **Wt gms** | **%w/w** | **% w/w** |
| Sugar (matrix) | 360 | 18.0 | 33.4 |
| Gum acacia (stabilizer) | 360 | 18.0 | 33.4 |
| Egg yolk powder (emulsifier) | 5 | 0.25 | 0.47 |
| Acerola powder (anti-oxidant) | 10 | 0.5 | 0.93 |
| Citric acid (acidulent) | 5 | 0.25 | 0.46 |
| Mixed tocopherols (anti-oxidant) | 8 | 0.4 | 0.74 |
| Natural betacarotene (carotenoid) | 75 | 3.75 | 6.96 |
| Oil | 175 | 8.75 | 16.24 |
| Water | **1002** | **50.1** | **7.4** |
| | 2000g | 100 | 100 |

The dried product is the emulsion with approximately 92% of the water removed.

The composition of the invention is naturally derived as specified above and therefore is more acceptable as a food additive. Its preparation involves the formation of the carotenoid in an oil phase using a natural oil solvent, the dispersion of this into the aqueous phase of water dispersible matrix and stabilizer followed by emulsification. This emulsion can then be optionally spray dried to form a dried powder.

## Claims

1. A carotenoid composition comprising:
(a) a carotenoid in an oil solvent;
(b) a dispersion of a water-dispersible matrix and a stabiliser and optionally a non-oil solvent; and
(c) an emulsifier
and which composition is substantially free of synthetic or chemically-modified components and wherein the weight of the carotenoid in the carotenoid composition when dried is from 0.5 up to 12 wt %.

2. A carotenoid composition as claimed in claim 1, wherein the weight of the carotenoid in the composition is in the range of 7.5% up to 12%.

3. A carotenoid composition as claimed in claim 1 or claim 2, wherein the composition is in the form of a powder.

4. A carotenoid composition as claimed in claim 1, wherein the weight of the carotenoid in the composition is up to 7.5%.

5. A carotenoid composition as claimed in claim 1 or claim 4, wherein the composition is in the form of a liquid.

6. A carotenoid composition as claimed in any of claims 1, 4 or 5, wherein component (b) comprises a non-oil solvent.

7. A carotenoid composition as claimed in any of claims 1, 4, 5 or 6, wherein the non-oil solvent is selected from water, glycerol and mixtures thereof.

8. A carotenoid composition as claimed in any preceding claim, wherein the water-dispersible matrix of component (b) is initially formed from a non-oil solvent and a non-oil solvent-soluble matrix and a non-oil solvent soluble anti-oxidant.

9. A carotenoid composition as claimed in any preceding claim wherein the matrix is formed from a combination of any one or more of sucrose, fructose, glucose, mannose, pentoses, maltose and malto dextrins.

10. A carotenoid composition as claimed in claim 6, wherein the composition comprises a non-oil solvent-soluble anti-oxidant.

11. A carotenoid composition as claimed in claim 10, wherein the non-oil solvent-soluble anti-oxidant is natural ascorbic acid.

12. A carotenoid composition as claimed in claim 11, wherein the anti-oxidant is derived from rosehip and/or acerola.

13. A carotenoid composition as claimed in any of the preceding claims, wherein the oil solvent contains an oil-soluble anti-oxidant at 0.1 to 2% of the carotenoid composition

14. A carotenoid composition as claimed in claim 13, wherein the oil-soluble anti-oxidant is a combination of natural tocopherols and other plant extracts having anti-oxidant activity, and mixtures thereof.

15. A carotenoid composition as claimed in any of the preceding claims, wherein the carotenoid is selected from fat soluble retinoids, beta-carotene, lutein, lycopene, astaxanthin, canthaxanthin, phytoene, alpha-carotene, apo carotenal, retinol, capsanthin, oleo resin paprika, zeaxanthin, betacryptoxanthin, phytofluene, gamma-carotene, natural carotenoid isolates and sources, and mixtures thereof.

16. A carotenoid composition as claimed in any preceding claim, wherein the carotenoid is beta-carotene.

17. A carotenoid composition as claimed in any of the preceding claims, wherein the oil solvent is selected from animal, vegetable or mineral oils, and mixtures thereof.

18. A carotenoid composition as claimed in any of the preceding claims, wherein the oil solvent is selected from soybean oil, peanut oil, lard, olive oil, corn oil, coconut oil, fish oil, terpenoid and essential oils, and mixtures thereof.

19. A carotenoid composition as claimed in any of the preceding claims, wherein the emulsifier is selected from extracts of animal origin, plant extracts, phospholipid extracts of natural products, saponins, milk isolates and egg products, and mixtures thereof.

20. A carotenoid composition as claimed in any of the preceding claims, wherein the stabiliser is selected from hydrocolloids derived from natural plant or animal sources; guar, xanthan, carob, karaya, arabic, acacia and furcellaran gums; and carageenans, alginates, celluloses, gelatins, starches, amylose and amylopectin; and mixtures thereof.

21. A carotenoid composition as claimed in any of the preceding claims, wherein the composition additionally comprises an acidulant that either exists naturally or is derived from natural sources.

22. A carotenoid composition as claimed in any of claims 1 to 4 and 6 to 21, wherein the water dispersible matrix comprises up to 90% by weight of the composition in a dried form.

23. A carotenoid composition as claimed in any of claims 1 to 22, wherein the composition is substantially homogenous.

24. A process for producing a carotenoid composition, the process comprising the following steps:
(a) forming an oil phase by dispersing a carotenoid in an oil solvent;
(b) forming an aqueous phase by dispersing a water dispersible matrix, a stabiliser and a non-oil solvent;
(c) admixing the oil phase and the aqueous phase; and
(d) emulsifying the admixture with an emulsifier,
provided that the carotenoid composition so formed is substantially free of synthetic or chemically-modified components.

25. A process according to claim 24, wherein the water dispersible matrix is prepared by dispersing a non-oil solvent-soluble matrix-forming element in a non-oil solvent.

26. A process for producing a carotenoid composition as claimed in claim 24 or claim 25, further comprising after step (d), the step of homogenising the emulsion to obtain a fine particle structure.

27. A process for producing a carotenoid composition as claimed in claim 26, wherein the fine particle structure is within 0.01 and 10 microns.

28. A process for producing a carotenoid composition, as claimed in any one of claims 24 to 27, further comprising the step of adding an acidulant to the aqueous phase to adjust the pH of the aqueous phase to within the range of 2 to 5.5.

29. A process for producing a carotenoid composition as claimed in any one of claims 24 to 28, further comprising between step (c) and step (d), the step of heating the admixture to a temperature of between 60°C and 150°C.

30. A process for producing a carotenoid composition as claimed in any one of claims 24 to 29, further comprising the step of drying the resultant emulsion.

31. A process for producing a carotenoid composition as claimed in claim 30, wherein the emulsion is dried using a spray drier.

32. A carotenoid composition obtainable by the process as claimed in any one of claims 24 to 31.

## Revendications

1. Composition caroténoïde comprenant :
(a) un caroténoïde dans un solvant huileux ;
(b) une dispersion d'une matrice dispersible dans l'eau et un stabilisateur et éventuellement un solvant non huileux ; et
(c) un émulsifiant
cette composition étant dans l'ensemble libre de constituants synthétiques ou modifiés chimiquement, et le poids de caroténoïde dans la composition caroténoïde une fois séchée va de 0,5 Jusqu'à 12 % en poids.

2. Composition caroténoïde selon la revendication 1,
**caractérisée en ce que**
le poids de caroténoïde dans la composition se trouve dans la gamme allant de 7,5 % jusqu'à 12 %.

3. Composition caroténoïde selon la revendication 1 ou la revendication 2,
**caractérisée en ce que**
la composition est sous forme d'une poudre.

4. Composition caroténoïde selon la revendication 1,
**caractérisée en ce que**
le poids de caroténoïde dans la composition va jusqu'à 7,5 %.

5. Composition caroténoïde selon la revendication 1 ou la revendication 4,
**caractérisée en ce que**
la composition est sous forme liquide.

6. Composition caroténoïde selon l'une quelconque des revendications 1, 4 ou 5,
**caractérisée en ce que**
le constituant (b) comprend un solvant non huileux.

7. Composition caroténoïde selon l'une quelconque des revendications 1, 4, 5 ou 6,
**caractérisée en ce que**
le solvant non huileux est choisi à partir d'eau, de glycérol et de leurs mélanges.

8. Composition caroténoïde selon l'une quelconque des revendications précédentes,
**caractérisée en ce que**
la matrice dispersible dans l'eau du constituant (b) est initialement formée à partir d'un solvant non huileux et d'une matrice soluble dans un solvant non huileux et d'un antioxydant soluble dans un solvant non huileux.

9. Composition caroténoïde selon l'une quelconque des revendications précédentes,
**caractérisée en ce que**
la matrice est formée à partir d'une combinaison de n'importe lequel ou de plusieurs des saccharoses, fructose, glucose, mannose, pentoses, maltose et maltodextrines.

10. Composition caroténoïde selon la revendication 6,
**caractérisée en ce que**
la composition comprend un antioxydant soluble dans un solvant non huileux.

11. Composition caroténoïde selon la revendication 10,
**caractérisée en ce que**
l'antioxydant soluble dans un solvant non huileux est de l'acide ascorbique naturel.

12. Composition caroténoïde selon la revendication 11,
**caractérisée en ce que**
l'antioxydant provient d'églantine et/ou d'acérola.

13. Composition caroténoïde selon l'une quelconque des revendications précédentes,
**caractérisée en ce que**
le solvant huileux contient un antioxydant soluble dans l'huile allant de 0,1 à 2 % de la composition caroténoïde.

14. Composition caroténoïde selon la revendication 13,
**caractérisée en ce que**
l'antioxydant soluble dans l'huile est un combinaison de tocophérols naturels et d'autres extraits de plantes ayant une activité anti-oxydante, et leurs mélanges.

15. Composition caroténoïde selon l'une quelconque des revendications précédentes,
**caractérisée en ce que**
le caroténoïde est choisi parmi les retinoïdes solubles dans les graisses, le béta-carotène, la lutéïne, le lycopène, l'astaxanthine, la canthaxanthine, le phytone, l'alpha-carotène, l'apo-caroténal, le rétinol, la capsanthine, le paprika d'oléorésine, la zéaxanthine, la bétacryptoxanthine, le phytofluène, le gamma-carotène, les isolats de caroténoïdes naturels et les sources d'origine, et leurs mélanges.

16. Composition caroténoïde selon l'une quelconque des revendications précédentes,
**caractérisée en ce que**
le caroténoïde est le béta-carotène.

17. Composition caroténoïde selon l'une quelconque des revendications précédentes,
**caractérisée en ce que**
le solvant huileux est choisi parmi les huiles animales, végétales ou minérales, et leurs mélanges.

18. Composition caroténoïde selon l'une quelconque des revendications précédentes,
**caractérisée en ce que**
le solvant huileux est choisi parmi l'huile de graine de soja, l'huile de cacahuète, le saindoux, l'huile d'olive, l'huile de maïs, l'huile de noix de coco, l'huile de poisson, les huiles terpénoïdes et essentielles, et leurs mélanges.

19. Composition caroténoïde selon l'une quelconque des revendications précédentes,
**caractérisée en ce que**
l'émulsifiant est choisi parmi les extraits d'origine animale, les extraits de plantes, les extraits de phospholipides des produits naturels, les saponines, les isolats de lait et les produits des oeufs, et leurs mélanges.

20. Composition caroténoïde selon l'une quelconque des revendications précédentes,
**caractérisée en ce que**
le stabilisateur est choisi parmi les hydrocolloïdes provenant de sources naturelles de plantes ou d'animaux ; les gommes de guar, de xanthane, de caroubier, de sterculia, d'arabique, d'acacia et de furcellaran ; et les caragénanes, les alginates, les celluloses, les gélatines, les amidons, l'amylose et l'amilopectine ; et leurs mélanges.

21. Composition caroténoïde selon l'une quelconque des revendications précédentes,
**caractérisée en ce que**
la composition comprend en plus un acidulant qui soit existe naturellement soit provient de sources naturelles.

22. Composition caroténoïde selon l'une quelconque des revendications 1 à 4 et 6 à 21,
**caractérisée en ce que**
la matrice dispersible dans l'eau constitue Jusqu'à 90 % en poids de la composition sous une forme séchée.

23. Composition caroténoïde selon l'une quelconque des revendications 1 à 22,
**caractérisée en ce que**
la composition est dans l'ensemble homogène.

24. Procédé pour produire une composition caroténoïde, le procédé comprenant les étapes suivantes :
(a) former une phase huileuse en dispersant un caroténoïde dans un solvant huileux ;
(b) former une phase aqueuse en dispersant une matrice dispersible dans l'eau, un stabilisateur et un solvant non huileux ;
(c) mélanger la phase huileuse et la phase aqueuse ; et
(d) émulsionner le mélange avec un émulsiflant,
à condition que la composition caroténoïde ainsi formée soit dans l'ensemble libre de constituants synthétiques ou modifiés chimiquement.

25. Procédé selon la revendication 24,
**caractérisé en ce que**
la matrice dispersible dans l'eau est préparée en dispersant dans un solvant non huileux, un élément huileux formant la matrice et soluble dans un solvant non huileux.

26. Procédé pour produire une composition caroténoïde selon la revendication 24 ou la revendication 25, comprenant en plus après l'étape (d), l'étape d'homogénéisation de l'émulsion pour obtenir une structure en particules fines.

27. Procédé pour produire une composition caroténoïde selon la revendication 26,
**caractérisé en ce que**
la structure en particules fines est entre 0,01 et 10 microns.

28. Procédé pour produire une composition caroténoïde selon l'une quelconque des revendications 24 à 27, comprenant de plus l'étape d'ajouter un acidulant à la phase aqueuse pour ajuster le pH de la phase aqueuse dans la gamme allant de 2 à 5,5.

29. Procédé pour produire une composition caroténoïde selon l'une quelconque des revendications 24 à 28, comprenant de plus entre l'étape (c) et l'étape (d), l'étape de chauffer le mélange à une température entre 60°C et 150°C.

30. Procédé pour produire une composition caroténoïde selon l'une quelconque des revendications 24 à 29, comprenant
de plus l'étape de sécher l'émulsion résultante.

31. Procédé pour produire une composition caroténoïde selon la revendication 30.
**caractérisé en ce que**
l'émulsion est séchée en utilisant un sécheur par pulvérisation.

32. Composition caroténoïde susceptible d'être obtenue par le procédé tel que revendiqué selon l'une quelconque des revendications 24 à 31.

## Patentansprüche

1. Carotinoidzusammensetzung, umfassend:
(a) ein Carotinoid in einem Öllösungsmittel;
(b) eine Dispersion aus einer wasserdispergierbaren Matrix und einem Stabilisator und gegebenenfalls einem Nicht-Öllösungsmittel; und
(c) einen Emulgator;
wobei die Zusammensetzung im wesentlichen frei von synthetischen oder chemisch modifizierten Komponenten ist und wobei das Gewicht des Carotinoids in der Carotinoidzusammensetzung im getrockneten Zustand 0,5 bis zu 12 Gew.-% beträgt.

2. Carotinoidzusammensetzung gemäß Anspruch 1, wobei das Gewicht des Carotinoids in der Zusammensetzung im Bereich von 7,5% bis zu 12% liegt.

3. Carotinoidzusammensetzung gemäß Anspruch 1 oder 2, wobei die Zusammensetzung in Form eines Pulvers vorliegt.

4. Carotinoidzusammensetzung gemäß Anspruch 1, wobei das Gewicht des Carotinoids in der Zusammensetzung bis zu 7,5% beträgt.

5. Carotinoidzusammensetzung gemäß Anspruch 1 oder 4, wobei die Zusammensetzung in Form einer Flüssigkeit vorliegt.

6. Carotinoidzusammensetzung gemäß einem der Ansprüche 1, 4 oder 5,
wobei Komponente (b) ein Nicht-Öllösungsmittel umfasst.

7. Carotinoidzusammensetzung gemäß einem der Ansprüche 1, 4, 5 oder 6,
wobei das Nicht-Öllösungsmittel aus Wasser, Glycerin und Gemischen davon ausgewählt ist.

8. Carotinoidzusammensetzung gemäß einem der vorstehenden Ansprüche,
wobei die wasserdispergierbare Matrix von Komponente (b) anfangs aus einem Nicht-Öllösungsmittel und einer in einem Nicht-Öllösungsmittel löslichen Matrix und einem in einem Nicht-Öllösungsmittel löslichen Antioxidans besteht.

9. Carotinoidzusammensetzung gemäß einem der vorstehenden Ansprüche,
wobei die Matrix aus einer Kombination von einem oder mehreren der Substanzen Saccharose, Fructose, Glucose, Mannose, Pentosen, Maltose und Maltodextrine besteht.

10. Carotinoidzusammensetzung gemäß Anspruch 6, wobei die Zusammensetzung ein in einem Nicht-Öllösungsmittel lösliches Antioxidans umfasst.

11. Carotinoidzusammensetzung gemäß Anspruch 10, wobei es sich bei dem in einem Nicht-Öllösungsmittel löslichen Antioxidans um natürliche Ascorbinsäure handelt.

12. Carotinoidzusammensetzung gemäß Anspruch 11, wobei das Antioxidans von Hagebutten und/oder Barbadoskirsche abgeleitet ist.

13. Carotinoidzusammensetzung gemäß einem der vorstehenden Ansprüche,
wobei das Öllösungsmittel ein öllösliches Antioxidans in einer Menge von 0,1 bis 2% der Carotinoidzusammensetzung enthält.

14. Carotinoidzusammensetzung gemäß Anspruch 13, wobei es sich bei dem öllöslichen Antioxidans um eine Kombination von natürlichen Tocopherolen und anderen Pflanzenextrakten mit Antioxidanswirkung oder Gemischen davon handelt.

15. Carotinoidzusammensetzung gemäß einem der vorstehenden Ansprüche,
wobei das Carotinoid aus fettlöslichen Retinoiden, β-Carotin, Lutein, Lycopen, Astaxanthin, Canthaxanthin, Phytoen, α-Carotin, apo-Carotinal, Retinol, Capsanthin, Ölharzpaprika, Zeaxanthin, β-Cryptoxanthin, Phytofluen, γ-Carotin, natürlichen Carotinoid-Isolaten und -Quellen und Gemischen davon ausgewählt ist.

16. Carotinoidzusammensetzung gemäß einem der vorstehenden Ansprüche,
wobei es sich bei dem Carotinoid um β-Carotin handelt.

17. Carotinoidzusammensetzung gemäß einem der vorstehenden Ansprüche,
wobei das Öllösungsmittel aus tierischen, pflanzlichen oder Mineralölen sowie Gemischen davon ausgewählt ist.

18. Carotinoidzusammensetzung gemäß einem der vorstehenden Ansprüche,
wobei das Öllösungsmittel aus Sojaöl, Erdnussöl, Schmalz, Olivenöl, Maiskeimöl, Kokosnussöl, Fischöl, Terpenoiden und ätherischen Ölen sowie Gemischen davon ausgewählt ist.

19. Carotinoidzusammensetzung gemäß einem der vorstehenden Ansprüche,
wobei der Emulgator aus Extrakten tierischer Herkunft, Pflanzenextrakten, Phospholipidextrakten von natürlichen Produkten, Saponinen, Milchisolaten und Eierprodukten sowie Gemischen davon ausgewählt ist.

20. Carotinoidzusammensetzung gemäß einem der vorstehenden Ansprüche,
wobei der Stabilisator aus Hydrokolloiden, die von natürlichen pflanzlichen oder tierischen Quellen stammen, Guar-, Xanthan-, Johannisbrot-, Karaya-, Arabin-, Akazien- und Furcellarangummi sowie Carrageenen, Alginaten, Cellulosen, Gelatinen, Stärken, Amylose und Amylopectin sowie Gemischen davon ausgewählt ist.

21. Carotinoidzusammensetzung gemäß einem der vorstehenden Ansprüche,
wobei die Zusammensetzung zusätzlich ein Säuerungsmittel umfasst, dass entweder natürlich vorkommt oder von natürlichen Quellen abgeleitet ist.

22. Carotinoidzusammensetzung gemäß einem der Ansprüche 1 bis 4 und 6 bis 21, wobei die wasserdispergierbare Matrix bis zu 90 Gew.-% der Zusammensetzung in einer getrockneten Form umfasst.

23. Carotinoidzusammensetzung gemäß einem der Ansprüche 1 bis 22, wobei die Zusammensetzung im wesentlichen homogen ist.

24. Verfahren zur Herstellung einer Carotinoidzusammensetzung, wobei das Verfahren die folgenden Schritte umfasst:
(a) Bilden einer Ölphase durch Dispergieren eines Carotinoids in einem Öllösungsmittel;
(b) Bilden einer wässrigen Phase durch Dispergieren einer wasserdispergierbaren Matrix, eines Stabilisators und eines Nicht-Öllösungsmittels;
(c) Mischen der Ölphase und der wässrigen Phase; und
(d) Emulgieren des Gemischs mit einem Emulgator;
mit der Maßgabe, dass die so gebildete Carotinoidzusammensetzung im wesentlichen frei von synthetischen oder chemisch modifizierten Komponenten ist.

25. Verfahren gemäß Anspruch 24, wobei die wasserdispergierbare Matrix hergestellt wird, indem man ein Element, das eine in einem Nicht-Öllösungsmittel lösliche Matrix bildet, in einem Nicht-Öllösungsmittel löst.

26. Verfahren zur Herstellung einer Carotinoidzusammensetzung gemäß Anspruch 24 oder 25, das weiterhin nach Schritt (d) den Schritt des Homogenisierens der Emulsion umfasst, so dass man eine feinteilige Struktur erhält.

27. Verfahren zur Herstellung einer Carotinoidzusammensetzung gemäß Anspruch 26, wobei sich die feinteilige Struktur zwischen 0,01 und 10 um bewegt.

28. Verfahren zur Herstellung einer Carotinoidzusammensetzung gemäß einem der Ansprüche 24 bis 27, das weiterhin den Schritt des Hinzufügens eines Säuerungsmittels zu der wässrigen Phase umfasst, so dass der pH-Wert der wässrigen Phase auf einen Wert im Bereich von 2 bis 5,5 eingestellt wird.

29. Verfahren zur Herstellung einer Carotinoidzusammensetzung gemäß einem der Ansprüche 24 bis 28, das weiterhin zwischen Schritt (c) und Schritt (d) den Schritt des Erhitzens des Gemischs auf eine Temperatur zwischen 60 °C und 150 °C umfasst.

30. Verfahren zur Herstellung einer Carotinoidzusammensetzung gemäß einem der Ansprüche 24 bis 29, das weiterhin den Schritt des Trocknens der resultierenden Emulsion umfasst.

31. Verfahren zur Herstellung einer Carotinoidzusammensetzung gemäß Anspruch 30, wobei die Emulsion unter Verwendung eines Sprühtrockners getrocknet wird.

32. Carotinoidzusammensetzung, die nach dem Verfahren gemäß einem der Ansprüche 24 bis 31 erhältlich ist.
